(19) Europäisches Patentamt
European Patent Office
Office européen des brevets

(11) **EP 3 959 515 B1**

(12) **EUROPÄISCHE PATENTSCHRIFT**

(45) Veröffentlichungstag und Bekanntmachung des Hinweises auf die Patenterteilung:
**19.02.2025  Patentblatt 2025/08**

(21) Anmeldenummer: **20716434.4**

(22) Anmeldetag: **31.03.2020**

(51) Internationale Patentklassifikation (IPC):
*G01N 29/024* (2006.01)  *G01N 29/036* (2006.01)
*G01N 29/22* (2006.01)  *G01N 29/44* (2006.01)
*C12C 7/04* (2006.01)  *C12C 7/06* (2006.01)
*G01N 9/36* (2006.01)  *G01N 33/14* (2006.01)
*G01N 9/24* (2006.01)

(52) Gemeinsame Patentklassifikation (CPC):
**G01N 29/4436; C12C 7/04; C12C 7/06; G01N 9/002; G01N 9/36; G01N 29/024; G01N 29/036; G01N 29/222; G01N 33/143; G01N 33/146;** G01N 9/24; G01N 2009/006; G01N 2291/022; G01N 2291/024; G01N 2291/02809

(86) Internationale Anmeldenummer:
**PCT/EP2020/059048**

(87) Internationale Veröffentlichungsnummer:
**WO 2020/216582 (29.10.2020 Gazette 2020/44)**

(54) **VERFAHREN ZUR BESTIMMUNG UND/ODER ÜBERWACHUNG EINER KONZENTRATION VON MALTODEXTRIN UND/ODER MALTOSE IN EINEM MAISCHPROZESS**

METHOD FOR DETERMINING AND/OR MONITORING A CONCENTRATION OF MALTODEXTRIN AND/OR MALTOSE IN A MASHING PROCESS

MÉTHODE POUR DÉTERMINER ET/OU SURVEILLER UNE CONCENTRATION DE MALTODEXTRINE OU DE MALTOSE DANS UN PROCESSUS DE MACÉRATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.04.2019  DE 102019110821**

(43) Veröffentlichungstag der Anmeldung:
**02.03.2022  Patentblatt 2022/09**

(73) Patentinhaber: **Endress+Hauser SE+Co. KG**
**79689 Maulburg (DE)**

(72) Erfinder:
• **LOPATIN, Sergey**
**79540 Lörrach (DE)**

• **ROSENHEIM, Julia**
**79664 Wehr (DE)**

(74) Vertreter: **Koslowski, Christine Adelheid**
**Endress+Hauser Group Services (Deutschland) AG+Co. KG**
**Colmarer Straße 6**
**79576 Weil am Rhein (DE)**

(56) Entgegenhaltungen:
EP-B1- 3 129 460    CA-A1- 2 837 317
DE-A1- 10 035 624    DE-A1- 102016 109 250
DE-A1- 4 023 977    DE-A1- 4 437 684
US-A- 5 359 541

**Beschreibung**

Die Erfindung betrifft ein Verfahren zur fortlaufenden Bestimmung und/oder Überwachung einer Konzentration von Maltodextrin und/oder Maltose in einem Maischprozess. Das Verfahren kann beispielsweise mittels einer Vorrichtung umfassend eine Sensoreinheit mit zumindest einer mechanisch schwingfähigen Einheit und zumindest einem piezoelektrischen Element durchgeführt werden.

Für eine zuverlässige Überwachung komplexer Prozesse ist die simultane Kenntnis unterschiedlichster Prozessgrößen erforderlich. Ein Beispiel für einen solchen Prozess ist ein Maischprozess, der beispielsweise beim Brauen von Bier angewendet wird. Dabei wird beispielsweise Malzschrot, welches aus Getreide, insbesondere Gerste, hergestellt wird, in Wasser eingeweicht und erwärmt. Bei diesem Vorgang werden verschiedene Malzinhaltsstoffe, wie Stärken, Eiweiße oder auch Zellwandsubstanzen, durch verschiedene enzymatische, physikalische und chemische Vorgänge gelöst. Beispielsweise spalten $\alpha$- und $\beta$-Amylasen verschiedene Stärken, insbesondere Amylose und Amylopektin im Malzschrot zu Maltose, Dextrin und Glucose; Gluco-Amylasen dagegen spalten D-Maltose zu D-Glucose.

Grundsätzlich wird beim Maischen versucht, die Temperatur derart einzustellen, dass ein hoher Verzuckerungsgrad der Stärke in Maltose erreicht wird. Deshalb wird üblicherweise in einem für die $\alpha$- und $\beta$-Amylasen optimalen Temperaturbereich gearbeitet. Die optimale Temperatur für $\alpha$-Amylase liegt bei T<70°C, die für $\beta$-Amylase bei T$\approx$75°C, so dass nacheinander in zwei Arbeitsschritten zwei unterschiedliche Temperaturen eingestellt werden müssen, um einen maximal möglichen Verzuckerungsgrad zu erreichen. In vielen Fällen wird das Vorhandensein von löslicher Amylose während und am Ende des Maischprozesses über eine refraktometrische Messung des Brechungsindex oder über eine manuelle Jodprobe durchgeführt. Die zur Verzuckerung benötigte Zeit wird dabei in der Regel empirisch festgelegt und nicht an die tatsächlichen Verläufe angepasst. Es wäre wünschenswert, die Zeitdauern für die einzelnen Arbeitsschritte genau bestimmen zu können.

Die DE4437684A1 beschreibt eine Vorrichtung zur Messung der Konzentration einer in einem Behältnis befindlichen Flüssigkeit wie gärende Würze bei der Bierherstellung.

Die DE4023977A1 beschreibt ein Verfahren zur Kontrolle und Steuerung der Konzentration von Suspensionen, Emulsionen und Lösungen.

Die DE10035624A1 beschreibt eine Vorrichtung und Verfahren zur Fluiddiagnose.

Die US5359541A beschreibt die Bestimmung einer Lösungskonzentration einer Flüssigkeit in einem Prozess.

Die DE102016109250A1 beschreibt ein Verfahren zur Konzentrationsbestimmung von Komponenten in einem mehrkomponentigem Stoffgemisch.

Die EP 3 129 460 B1 beschreibt ein Verfahren zum Steuern eines Maischprozesses über das Auswerten von Infrarot-Spektren. Dabei werden Verhältnisse von unterschiedlichen Zuckertypen ermittelt. Weitere Größen ergeben sich durch die Modellierung der stattfindenden Abbauprozesse.

Der vorliegenden Erfindung liegt somit die Aufgabe zugrunde, eine einfache Möglichkeit zur Überwachung eines Verzuckerungsgrads in einem Maischprozess anzugeben.

Diese Aufgabe wird gelöst durch ein Verfahren mit den Merkmalen des ersten Anspruchs.

Vorteilhaft kann anhand der Dichte und der Schallgeschwindigkeit der Umwandlungsgrad von Maltodextrin in Maltose bestimmt werden. Durch fortlaufende Bestimmung der Konzentrationen von Maltodextrin und/oder Maltose in der Maische kann die Zeitdauer zur Verzuckerung der angestellten Maische exakt bestimmt werden. Hierdurch kann sowohl die Anlagenverfügbarkeit als auch die zum Maischen benötigte Energie sowie die benötigten Dampfmengen zum Heißhalten der Maische optimiert werden.

In einer Ausgestaltung des Verfahrens wird die Temperatur der Maische bestimmt. Sowohl die Dichte als auch die Schallgeschwindigkeit sind temperaturabhängige Größen.

So ist es von Vorteil, wenn ein Einfluss der Temperatur auf die Bestimmung der Dichte und/oder Schallgeschwindigkeit berücksichtigt wird. Der Einfluss der Temperatur kann insbesondere kompensiert werden. Hierdurch lassen sich die Konzentrationen der Maltose und/oder des Maltodextrins zuverlässiger bestimmen.

Erfindungsgemäß wird die Konzentration der Summe von Maltodextrin und Maltose in der Maische anhand der Dichte ermittelt. Die Dichte gibt Aufschluss über die Konzentration, insbesondere die Gewichtskonzentration, der Summe aus Maltodextrin und Maltose in der Maische. Auf diese Weise kann also die Gesamtkonzentration der beiden Stoffe in der Maische ermittelt werden.

Erfindungsgemäß wird anhand der Schallgeschwindigkeit in der Maische das Verhältnis von Maltodextrin und Maltose ermittelt. Die Schallgeschwindigkeit in der Maische hängt vom Grad der Umwandlung von Maltodextrin in Maltose ab.

Es ist ferner von Vorteil, wenn zumindest ein Wert für die Schallgeschwindigkeit in der Maische mit zumindest einem Referenzwert oder mit einem Wert einer Kennlinie für die Schallgeschwindigkeit von Maltodextrin und/oder Maltose als Funktion der Konzentration von Maltodextrin und/oder Maltose verglichen wird.

In einer besonders bevorzugten Ausgestaltung des Verfahrens wird

- eine Sensoreinheit mittels eines Anregesignals zu mechanischen Schwingungen angeregt,
- die mechanischen Schwingungen von der Sensoreinheit empfangen und in ein erstes Empfangssignal umgewandelt,

- von der Sensoreinheit ein Sendesignal ausgesendet und ein zweites Empfangssignal empfangen, und
- anhand des ersten Empfangssignals die Dichte und anhand des zweiten Empfangssignals die Schallgeschwindigkeit ermittelt.

Eine Vorrichtung, welche zur Durchführung eines erfindungsgemäßen Verfahrens geeignet ist, ist beispielsweise gegeben durch einen vibronischen Sensor. Vibronische Sensoren finden vielfach Anwendung in der Prozess- und/oder Automatisierungstechnik. Im Falle von Füllstandsmessgeräten weisen sie zumindest eine mechanisch schwingfähige Einheit, wie beispielsweise eine Schwinggabel, einen Einstab oder eine Membran auf. Diese wird im Betrieb mittels einer Antriebs-/Empfangseinheit, häufig in Form einer elektromechanischen Wandlereinheit, zu mechanischen Schwingungen angeregt, welche wiederum beispielsweise ein piezoelektrischer Antrieb oder ein elektromagnetischer Antrieb sein kann. Entsprechende Feldgeräte werden von der Anmelderin in großer Vielfalt hergestellt und beispielsweise unter der Bezeichnung LIQUIPHANT oder SOLIPHANT vertrieben. Die zugrundeliegenden Messprinzipien sind im Prinzip aus einer Vielzahl von Veröffentlichungen bekannt. Die Antriebs-/Empfangseinheit regt die mechanisch schwingfähige Einheit mittels eines elektrischen Anregesignals zu mechanischen Schwingungen an. Umgekehrt kann die Antriebs-/Empfangseinheit die mechanischen Schwingungen der mechanisch schwingfähigen Einheit empfangen und in ein elektrisches Empfangssignal umwandeln. Bei der Antriebs-/Empfangseinheit handelt es sich entsprechend entweder um eine separate Antriebseinheit und eine separate Empfangseinheit, oder um eine kombinierte Antriebs-/Empfangseinheit. Zur Anregung der mechanisch schwingfähigen Einheit zu mechanischen Schwingungen sind aus dem Stand der Technik unterschiedlichste Lösungen, sowohl analoge als auch digitale Verfahren, bekannt geworden, wie beispielsweise in den Dokumenten DE102006034105A1, DE102007013557A1, DE102005015547A1, DE102009026685A1, DE102009028022A1, DE102010030982A1 oder DE00102010030982A1 beschrieben.

Sowohl das Anregesignal als auch das Empfangssignal sind charakterisiert durch ihre Frequenz $\omega$, Amplitude A und/oder Phase $\Phi$. Entsprechend werden Änderungen in diesen Größen üblicherweise zur Bestimmung der jeweiligen Prozessgröße herangezogen. Bei der Prozessgröße kann es sich beispielsweise um einen Füllstand, einen vorgegebenen Füllstand, oder auch um die Dichte oder die Viskosität des Mediums, sowie um den Durchfluss handeln. Bei einem vibronischen Grenzstandschalters für Flüssigkeiten wird beispielsweise unterschieden, ob die schwingfähige Einheit von der Flüssigkeit bedeckt ist oder frei schwingt. Diese beiden Zustände, der Freizustand und der Bedecktzustand, werden dabei beispielsweise anhand unterschiedlicher Resonanzfrequenzen, also anhand einer Frequenzverschiebung, unterschieden. Die Dichte und/oder Viskosität wiederum lassen sich mit einem derartigen Messgerät nur ermitteln, wenn die schwingfähige Einheit vom Medium bedeckt ist. Im Zusammenhang mit der Bestimmung der Dichte und/oder Viskosität sind ebenfalls unterschiedliche Möglichkeiten aus dem Stand der Technik bekannt geworden, wie beispielswiese die in den Dokumenten DE10050299A1, DE102007043811A1, DE10057974A1, DE102006033819A1, DE102015102834A1 oder DE102016112743A1 offenbarten.

Darüber hinaus ist aus der bisher unveröffentlichten deutschen Patentanmeldung mit dem Aktenzeichen 102018127526.9 ein vibronischer Multisensor bekannt geworden, mittels welchem sowohl das vibronische Messprinzip als auch das Ultraschall-Messprinzip zur Bestimmung und/oder Überwachung einer oder mehrerer Prozessgrößen möglich ist. Mit einem derartigen Sensor kann sowohl die Dichte als auch die Schallgeschwindigkeit ermittelt werden. Verfügt der Sensor zusätzlich über einen Temperatursensor, so kann auch die Temperatur der Maische mittels desselben Messgeräts bestimmt werden.

Es sei aber darauf verwiesen, dass in anderen Ausgestaltungen die einzelnen Prozessgrößen aber auch zumindest teilweise von unterschiedlichen Messgeräten bestimmt werden können. Beispielsweise kann ein separater Temperatursensor zur Bestimmung der Temperatur herangezogen werden. Aber auch die Dichte und die Schallgeschwindigkeit können unabhängig voneinander ermittelt werden.

Die Erfindung wird anhand der nachfolgenden Figuren näher erläutert. Es zeigt:

Fig. 1: eine schematische Skizze eines vibronischen Sensors gemäß Stand der Technik,

Fig. 2 zwei mögliche an sich aus dem Stand der Technik bekannte Ausgestaltungen einer Sensoreinheit, welche zur Durchführung des erfindungsgemäßen Verfahrens geeignet sind, und

Fig. 3 eine Illustrierung einer möglichen Ausgestaltung des erfindungsgemäßen Verfahrens.

In den Figuren sind gleiche Elemente jeweils mit demselben Bezugszeichen versehen. Die nachfolgende Beschreibung bezieht sich ohne Beschränkung der Allgemeinheit auf den Fall, dass zur Durchführung des erfindungsgemäßen Verfahrens ein vibronischer Sensor 1 verwendet wird.

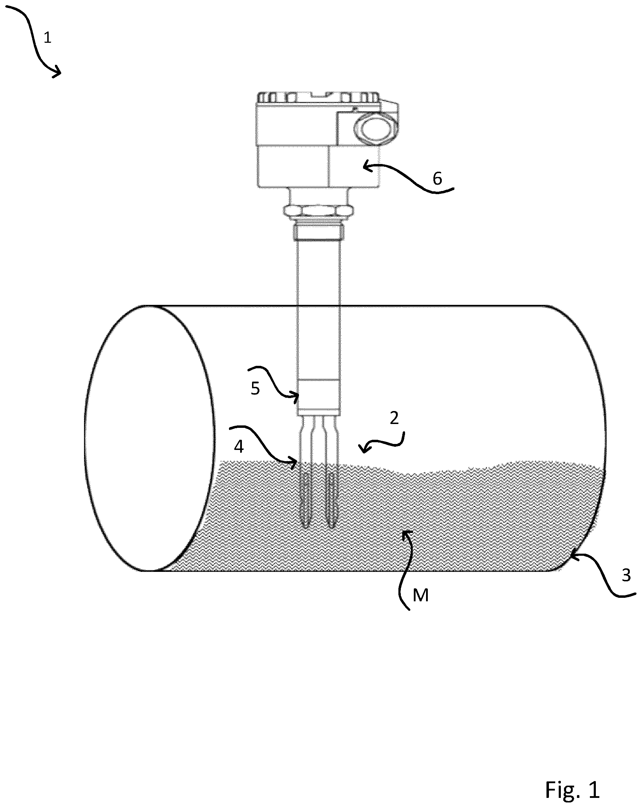

In Fig. 1 ist ein vibronischer Sensor 1 mit einer Sensoreinheit 2 gezeigt. Der Sensor verfügt über eine mechanisch schwingfähige Einheit 4 in Form einer Schwinggabel, welche teilweise in ein Medium M eintaucht, welches sich in einem Behälter 3 befindet. Die schwingfähige Einheit 4 wird mittels der Anrege-/Empfangseinheit 5 zu mechanischen Schwingungen angeregt, und kann beispielsweise durch einen piezoelektrischen Stapel- oder Bimorphantrieb sein. Andere vibronische Sensoren verfügen beispielsweise über elektromagnetische Antriebs-/Empfangseinheiten 5. Es ist sowohl möglich, eine einzige Antriebs-/Empfangseinheit 5 zu verwenden, welche zur Anregung der mechanischen Schwingungen sowie zu deren Detektion dient. Ebenso ist es aber denkbar, je eine Antriebseinheit und eine Empfangseinheit zu realisieren. Dargestellt ist in Fig. 1 ferner eine Elektronikeinheit 6, mittels welcher die Signalerfassung, -auswertung und/oder -speisung erfolgt.

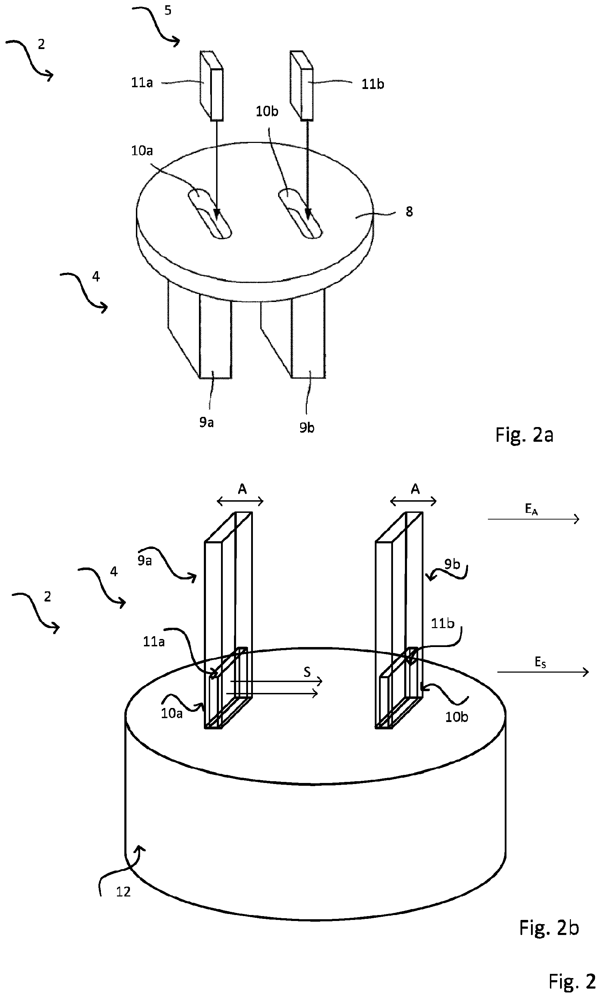

In Fig. 2a sind beispielhaft zwei verschiedene Sensoreinheiten 2 gezeigt, welche sich insbesondere zur Durchführung eines erfindungsgemäßen Verfahrens eignen. Die mechanisch schwingfähige Einheit 4 umfasst zwei an einer Basis 8 angebrachte Schwingelemente 9a,9b, welche mithin auch als Gabelzinken bezeichnet werden. Optional kann an den Endseiten der beiden Schwingelemente 9a,9b außerdem jeweils ein Paddel angeformt sein [hier nicht gezeigt]. In jeden der beiden Schwingelemente 9a,9b ist jeweils ein, insbesondere taschenartiger, Hohlraum 10a, 10b eingebracht, in welchem jeweils zumindest ein piezoelektrisches Element 11a, 11b der Antriebs-/Empfangseinheit 5 angeordnet ist. Vorzugsweise sind die piezoelektrischen Elemente 11a und 11b innerhalb der Hohlräume 10a und 10b vergossen. Die Hohlräume 10a, 10b können dabei so beschaffen sein, dass sich die beiden piezoelektrischen Elemente 11a, 11b vollständig oder teilweise im Bereich der beiden Schwingelemente 9a, 9b befinden Eine solche sowie ähnliche Anordnungen sind in der DE102012100728A1 ausführlich beschrieben.

Eine weitere beispielhafte, mögliche Ausgestaltung einer Sensoreinheit 2 ist in Fig. 2b dargestellt. Die mechanisch schwingfähige Einheit 4 verfügt über zwei parallel zueinander ausgerichtete, hier stabförmig ausgestaltete, auf einem scheibenförmigen Element 12 angebrachte, Schwingelemente 9a, 9b, welche getrennt voneinander zu mechanischen Schwingungen anregbar sind, und bei denen die Schwingungen ebenfalls getrennt voneinander empfangen und ausgewertet werden können. Beide Schwingelemente 9a und 9b weisen jeweils einen Hohlraum 10a und 10b auf, in welchen im dem scheibenförmigen Element 12 zugewandten Bereich jeweils zumindest ein piezoelektrisches Element 11a und 11b angeordnet ist. Bezüglich der Ausgestaltung gemäß Fig. 2b sei wiederum ferner auf in die bisher unveröffentlichte deutsche Patentanmeldung mit dem Aktenzeichen 102017130527.0 verwiesen.

Wie in Fig. 2b schematisch eingezeichnet, wird erfindungsgemäß die Sensoreinheit 2 einerseits mit einem Anregesignal A beaufschlagt, derart, dass die schwingfähige Einheit 4 zu mechanischen Schwingungen angeregt wird. Die Schwingungen werden dabei vermittels der beiden piezoelektrischen Elemente 11a und 11b erzeugt. Es ist sowohl denkbar, dass beide piezoelektrischen Elemente mit demselben Anregesignal A beaufschlagt werden, als auch eine Beaufschlagung des ersten Schwingelements 11a mittels eines ersten Anregesignals $A_1$ und des zweiten Schwingelements 11b mittels eines zweiten Anregesignals $A_2$. Ebenso ist es sowohl denkbar, dass anhand der mechanischen Schwingungen ein erstes Empfangssignal $E_A$, oder von jedem Schwingelement 9a,9b ein separates Empfangssignal $E_{A1}$ bzw. $E_{A2}$ empfangen wird.

Darüber hinaus wird vom ersten piezoelektrischen Element 11a ausgehend ein Sendesignal S ausgesendet, welches von dem zweiten piezoelektrischen Element 11b in Form eines zweiten Empfangssignals Es empfangen wird. Da die beiden piezoelektrischen Elemente 11a und 11b zumindest im Bereich der Schwingelemente 9a und 9b angeordnet sind, durchläuft das Sendesignal S das Medium M, sofern die Sensoreinheit 2 mit dem Medium M in Kontakt ist und wird entsprechend von den Eigenschaften des Mediums M beeinflusst. Vorzugsweise handelt es sich bei dem Sendesignal S um ein, insbesondere gepulstes, Ultraschallsignal, insbesondere um zumindest einen Ultraschallpuls. Ebenso ist es aber denkbar, dass das Sendesignal S von dem ersten piezoelektrischen Element 11a im Bereich des ersten Schwingelements 9a ausgesendet wird und an dem zweiten Schwingelement 9b reflektiert wird. In diesem Falle wird das zweite Empfangssignal Es vom ersten piezoelektrischen Element 11a empfangen. Das Sendesignal S durchläuft in diesem Falle das Medium M zweimal, was zu einer Verdoppelung einer Laufzeit $\tau$ des Sendesignals S führt und die Messgenauigkeit bezüglich der Bestimmung der Schallgeschwindigkeit erhöht.

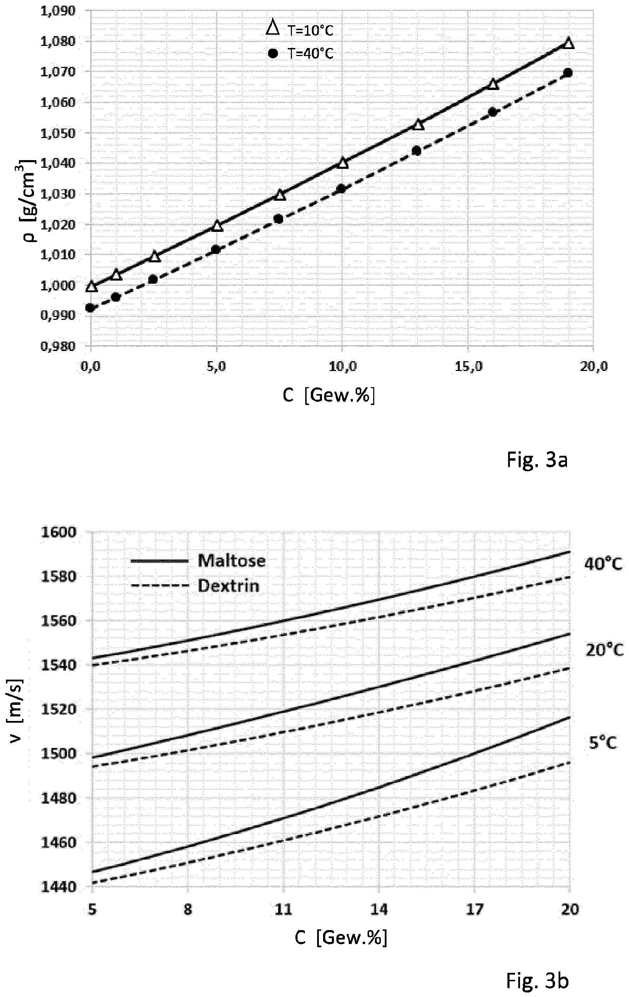

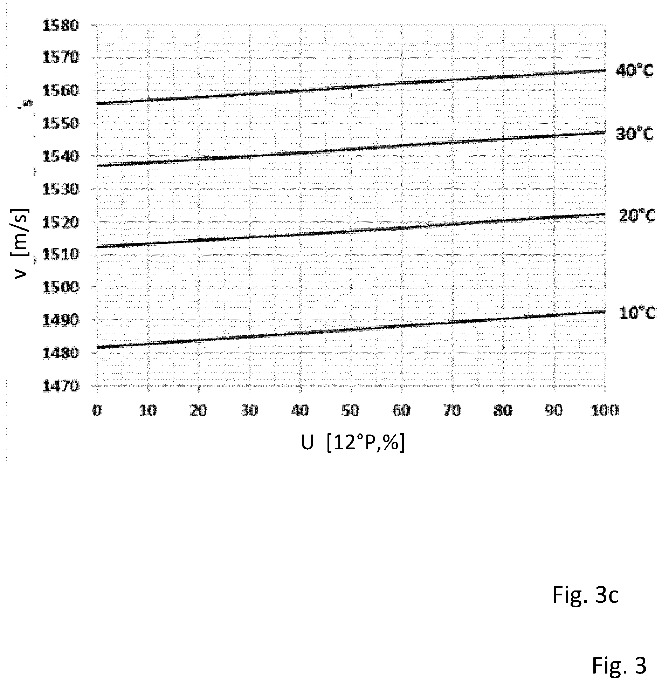

Das erste $E_A$ und zweite Empfangssignal Es resultieren aus unterschiedlichen Messverfahren und können unabhängig voneinander bezüglich unterschiedlicher Prozessgrößen $P_1$ und $P_2$, vorliegend der Dichte $\rho$ und die Schallgeschwindigkeit v, ausgewertet werden. Eine beispielhafte Ausgestaltung für das erfindungsgemäße Verfahren ist schließlich in Fig. 3 illustriert.

Fig. 3a zeigt die Dichte $\rho$ und Fig. 3b die Schallgeschwindigkeit v von Maltose und Dextrin in einer Maische für unterschiedliche Temperaturen T, jeweils als Funktion der Konzentration C. Die Dichte $\rho$ der Maische ist ein Maß für die Gesamtkonzentration von Maltose und Dextrin in der Maische. Die Dichte $\rho$ ist jedoch vom Umwandlungsgrad unabhängig. Dies gilt jedoch nicht für die Schallgeschwindigkeit v. Hier ergibt sich eine deutliche Abhängigkeit vom Verhältnis der Maltose und des Dextrins.

Weiterhin sind die Dichte $\rho$ und die Schallgeschwindigkeit v temperaturabhängig. Eine zusätzliche Bestimmung der Temperatur T der Maische ist demnach vorteilhaft.

Die Dichte $\rho$ kann im Falle der Verwendung eines in Fig. 2 beschriebenen vibronischen Sensors 1 beispielsweise anhand folgender Gleichung bestimmt werden:

$$\rho = \frac{1}{S} \cdot \left[ \left( \frac{F_0}{F_{Med}} \right)^2 - 1 \right]$$

Dabei ist $F_{Med}$ die Schwingungsfrequenz der schwingfähigen Einheit 4 im Medium M, $F_0$ die Referenzfrequenz der schwingfähigen Einheit 4 im Vakuum bzw. an Luft, und S beschreibt die Empfindlichkeit der Sensoreinheit 2. Die Schwingungsfrequenz der schwingfähigen Einheit 4 im Medium M $F_{Med}$ kann direkt anhand des ersten Empfangssignals $E_A$ ermittelt werden.

Die Schallgeschwindigkeit v des Mediums M lässt sich wiederum aus dem Abstand L zwischen dem ersten 11a und zweiten piezoelektrischen Element 11b, welche als Sendeeinheit und Empfangseinheit dienen, sowie der Laufzeit τ des Sendesignals S vom ersten 11a zum zweiten piezoelektrischen Element 11b nach folgender Gleichung ermitteln:

$$v = \frac{L}{\tau}$$

Zur Durchführung der hier beispielhaften gezeigten Ausgestaltung des erfindungsgemäßen Verfahrens wird anhand der Dichte ρ und der Temperatur T die Gesamtkonzentration von Maltose und Dextrin in der Maische ermittelt. Anschließend wird ein Wert für die Schallgeschwindigkeit bestimmt und mit den in Fig. 3c gezeigten Kennlinien für verglichen. Die Kennlinien zeigen hier beispielhaft die Schallgeschwindigkeit v in der Maische als Funktion des Umwandlungsgrads U, also des Verhältnisses von Maltose und Dextrin in der Maische für eine Plato-Konzentration von 12°P. Die Kennlinie, welche zum Vergleich jeweils heranzuziehen ist, wird also in Abhängigkeit der Gesamtkonzentration und in Abhängigkeit der Temperatur ausgewählt.

**Bezugszeichenliste**

| 1 | Vibronischer Sensor |
|---|---|
| 2 | Sensoreinheit |
| 3 | Behälter |
| 4 | Schwingfähige Einheit |
| 5 | Antriebs-/Empfangseinheit |
| 6 | Elektronikeinheit |
| 8 | Basis |
| 9a, 9b | Schwingelemente |
| 10a, 10b | Hohlräume |
| 11a, 11b | piezoelektrische Elemente |
| 12 | scheibenförmiges Element |

| M | Medium |
|---|---|
| $P_1$-$P_2$ | Prozessgrößen |
| A | Anregesignal |
| S | Sendesignal |
| $E_A$ | erstes Empfangssignal |
| Es | zweites Empfangssignal |
| ΔΦ | vorgebbare Phasenverschiebung |
| ρ | Dichte des Mediums |
| v | Schallgeschwindigkeit |
| τ | Laufzeit |
| T | Temperatur |

**Patentansprüche**

1. Verfahren zur fortlaufenden Bestimmung und/oder Überwachung einer Konzentration von Maltodextrin und/oder einer Konzentration von Maltose in einem Maischprozess,

    umfassend folgende Verfahrensschritte

        - Bereitstellen einer Maische,
        - Erwärmen der Maische auf zumindest eine vorgebbare Temperatur (T),
        - Bestimmen einer Dichte (p) der Maische unter Verwendung eines vibronischen Sensors (1) und
        - Bestimmen einer Schallgeschwindigkeit (v) in der Maische unter Verwendung des vibronischen Sensors (1),

    **dadurch gekennzeichnet,**

        - **dass** eine Konzentration der Summe von Maltodextrin und Maltose in der Maische anhand der bestimmten Dichte (p) der Maische ermittelt wird,
        - **dass** anhand der bestimmten Schallgeschwindigkeit (v) in der Maische ein Verhältnis von Maltodextrin und Maltose ermittelt wird, und
        - **dass** die Konzentration von Maltodextrin und/oder die Konzentration von Maltose in der Maische anhand des ermittelten Verhältnisses von Maltodextrin und Maltose ermittelt wird.

2. Verfahren nach Anspruch 1,

    wobei eine Temperatur (T) der Maische bestimmt wird,
    wobei die Konzentration der Summe von Maltodextrin und Maltose in der Maische anhand der bestimmten Dichte (p) und der bestimmten Temperatur (T) ermittelt wird, und
    wobei anhand der bestimmten Schallgeschwindigkeit (v) und der bestimmten Temperatur (T) das Verhältnis von Maltodextrin und Maltose ermittelt wird.

3. Verfahren nach Anspruch 1 oder 2,

wobei zumindest ein Wert für die Schallgeschwindigkeit (v) in der Maische mit zumindest einem Referenzwert oder mit einem Wert einer Kennlinie für die Schallgeschwindigkeit (v) von Maltodextrin und/oder Maltose als Funktion der Konzentration von Maltodextrin und/oder Maltose verglichen wird.

4. Verfahren nach einem der Ansprüche 1 bis 3, wobei

- eine Sensoreinheit (2) des vibronischen Sensors (1) mittels eines Anregesignals (A) zu mechanischen
Schwingungen angeregt wird,
- die mechanischen Schwingungen von der Sensoreinheit (2) empfangen und in ein erstes Empfangssignal ($E_A$) umgewandelt werden,
- von der Sensoreinheit (2) ein Sendesignal (S) ausgesendet und ein zweites Empfangssignal (Es) empfangen wird, und
- anhand des ersten Empfangssignals ($E_A$) die Dichte (p) und anhand des zweiten Empfangssignals (Es) die Schallgeschwindigkeit (v) ermittelt wird.

## Claims

1. Method for continuously determining and/or monitoring a concentration of maltodextrin and/or a concentration of maltose in a mashing process, comprising the following method steps

- Preparation of a mash,
- Heating the mash to at least a predetermined temperature (T),
- Determining a density (p) of the mash using a vibronic sensor (1) and
- Determine a sonic velocity (v) in the mash using the vibronic sensor (1),

**characterized in that**

- that a concentration of the sum of maltodextrin and maltose in the mash is determined on the basis of the determined density (p) of the mash,
- that a ratio of maltodextrin and maltose is determined on the basis of the determined sonic velocity (v) in the mash, and
- that the concentration of maltodextrin and/or the concentration of maltose in the mash is determined on the basis of the determined ratio of maltodextrin and maltose.

2. Method according to claim 1,
wherein a temperature (T) of the mash is determined, wherein the concentration of the sum of maltodextrin and maltose in the mash is determined on the basis of the determined density (p) and the determined temperature (T), and wherein the ratio of maltodextrin and maltose is determined on the basis of the determined sonic velocity (v) and the determined temperature (T).

3. Method according to claim 1 or 2,
wherein at least one value for the sonic velocity (v) in the mash is compared with at least one reference value or with a value of a characteristic curve for the sonic velocity (v) of maltodextrin and/or maltose as a function of the concentration of maltodextrin and/or maltose.

4. Method according to any one of claims 1 to 3, whereby

- a sensor unit (2) of the vibronic sensor (1) is excited to mechanical vibrations by means of an excitation signal (A)
- the mechanical vibrations are received by the sensor unit (2) and converted into a first received signal ($E_A$),
- a transmit signal (S) is transmitted by the sensor unit (2) and a second receive signal ($E_S$) is received, and
- the density (p) is determined using the first received signal ($E_A$) and the speed of sound (v) is determined using the second received signal ($E_S$).

## Revendications

1. Procédé de détermination et/ou de surveillance en continu d'une concentration de maltodextrine et/ou d'une concentration de maltose dans un processus d'empâtage, comprenant les étapes de procédé suivantes

- Préparation d'un moût,
- Chauffage du moût à au moins une température (T) pouvant être prédéfinie,
- déterminer une densité (p) du moût en utilisant un capteur vibronique (1) et
- déterminer une vitesse du son (v) dans le moût en utilisant le capteur vibronique (1),

**caractérisé en ce que**

- qu'une concentration de la somme de la maltodextrine et du maltose dans le moût est déterminée à l'aide de la densité déterminée (p) du moût,
- **en ce que**, à l'aide de la vitesse du son (v) déterminée dans le moût, on détermine un rapport de maltodextrine et de maltose, et
- **en ce que** la concentration de maltodextrine et/ou la concentration de maltose dans le moût est déterminée à l'aide du rapport déterminé de maltodextrine et de maltose.

2. Procédé selon la revendication 1, une température (T) du moût étant déterminée, la concentration de la somme de la maltodextrine et du maltose dans le moût étant déterminée à l'aide de la densité déterminée (p) et de la température déterminée (T), et le rapport de la maltodextrine et du maltose étant déterminé à l'aide de la vitesse du son déterminée (v) et de la température déterminée (T).

3. Procédé selon la revendication 1 ou 2, dans lequel au moins une valeur de la vitesse du son (v) dans le moût est comparée à au moins une valeur de référence ou à une valeur d'une courbe caractéristique de la vitesse du son (v) de la maltodextrine et/ou du maltose en fonction de la concentration de la maltodextrine et/ou du maltose.

4. Procédé selon l'une quelconque des revendications 1 à 3, où

   - une unité de détection (2) du capteur vibronique (1) est excitée en vibrations mécaniques au moyen d'un signal d'excitation (A)
   - les vibrations mécaniques sont reçues par l'unité de détection (2) et converties en un premier signal de réception ($E_A$),
   - un signal d'émission (S) est émis par l'unité de détection (2) et un deuxième signal de réception ($E_S$) est reçu, et
   - la densité (p) est déterminée à l'aide du premier signal de réception ($E_A$) et la vitesse du son (v) est déterminée à l'aide du deuxième signal de réception (Es).

Fig. 1

Fig. 2a

Fig. 2b

Fig. 2

Fig. 3a

Fig. 3b

Fig. 3c

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4437684 A1
- DE 4023977 A1
- DE 10035624 A1
- US 5359541 A
- DE 102016109250 A1
- EP 3129460 B1
- DE 102006034105 A1
- DE 102007013557 A1
- DE 102005015547 A1
- DE 102009026685 A1
- DE 102009028022 A1

- DE 102010030982 A1
- DE 00102010030982 A1
- DE 10050299 A1
- DE 102007043811 A1
- DE 10057974 A1
- DE 102006033819 A1
- DE 102015102834 A1
- DE 102016112743 A1
- DE 102018127526
- DE 102012100728 A1
- DE 102017130527